# EUROPÄISCHE PATENTSCHRIFT

(11) **EP 0 376 117 B1**
(45) Veröffentlichungstag und Bekanntmachung des Hinweises auf die Patenterteilung: **12.05.1993**
(21) Anmeldenummer: 89123421.3
(22) Anmeldetag: 19.12.1989
(51) Int. Cl.: C07C 57/04, C07C 51/25

(54) **Verfahren zur Herstellung von Methacrylsäure durch Oxidation von Methacrolein**
Preparation of methacrylic acid by the oxidation of methacrolein
Préparation d'acide méthacrylique par oxydation de méthacroléine

(30) Priorität: 28.12.1988 DE 3844087
(43) Veröffentlichungstag der Anmeldung: 04.07.1990
(73) Patentinhaber: BASF Aktiengesellschaft, 67063 Ludwigshafen (DE)
(72) Erfinder: Duembgen, Gerd, Dr., D-6700 Ludwigshafen (DE); Hammon, Ulrich, Dr., D-6710 Frankenthal (DE); Krabetz, Richard, Dr., D-6719 Kirchheim (DE); Schwarzmann, Matthias, Dr., D-6703 Limburgerhof (DE); Thiessen, Fritz, Dr., D-6700 Ludwigshafen (DE); Vogel, Herbert, Dr., D-6700 Ludwigshafen (DE)

(56) Entgegenhaltungen:
- EP-A- 0 043 100
- DE-A- 2 460 541
- DE-A- 3 010 434
- DE-A- 3 346 259
- US-A- 4 051 179

## Beschreibung

Die vorliegende Erfindung betrifft ein Verfahren zur Herstellung von Methacrylsäure durch Oxidation von Methacrolein in Sauerstoff und Wasserdampf enthaltenden Gasmischungen an Mo, P, V, Cs sowie As enthaltenden Katalysatoren.

Es ist bekannt, Methacrylsäure durch Oxidation von Methacrolein in Sauerstoff und Wasserdampf enthaltenden Gasmischungen in der Gasphase an molybdän- und phosphorhaltigen Katalysatoren herzustellen.

Als zusätzliche Katalysatorkomponente wird vielfach Arsen vorgeschlagen, doch ist der Arseneinfluß umstritten.

In der US-PS 3 998 876 wird ein Verfahren zur Herstellung von ungesättigten Carbonsäuren in Gegenwart eines Mo, P und As enthaltenden Katalysators beschrieben, der zusätzlich Elemente aus der Gruppe V, W, Cu, Fe, Mn, Sn und gegebenfalls Alkalimetalle enthält, die in Form eines Oxids, Nitrats oder Hydroxids eingesetzt werden müssen. Bezogen auf 12 Atome Mo soll der As-Gehalt 0.18 - 1.8 Atome, der Alkaligehalt 0 - 2.04 Atome, der P-Gehalt 0.36 - 2.4 Atome und der Gehalt der sonstigen Elemente 0.036 - 12 Atome betragen; außerdem sollen herstellungsbedingt NH₄-Gruppen in einer Menge von 0.12 - 2.4 Atomen vorhanden sein.

In der GB-PS 1 473 035 wird ein Verfahren mit Katalysatoren beschrieben, die neben Mo und P als zusätzliche Komponenten Cu, ein Alkalimetall, Elemente aus der Gruppe Sb, V, W, Fe, Mn, Sn und gegebenfalls As in Mengen von 0 bis weniger als 0.18 Atome je 12 Atome Mo enthalten. Es wird darauf hingewiesen, daß in solchen Katalysatoren sich das Arsen ungewöhnlich benimmt und außerhalb bestimmter Bereiche die Katalysatorleistung beeinträchtigt.

In der US-PS 4 596 784 wird ein Mo-Katalysator mit hohem Arsengehalt der Zusammensetzung

Mo₁₂P_{0.85-1.2}V_{0.05-0.7}As_{0.5-2}Cu_{0-0.4}Sb_{0-0.3}Cs/Tl/Rb_{0.75-1.25},

der ggf. noch andere Komponenten enthalten kann, beschrieben. Die Herstellung des Katalysators geht von MoO₃ aus und es wird darauf hingewiesen, daß der Einsatz anderer Mo-Quellen, z.B. Ammoniumheptamolybdat, zu niedrigen Katalysatorleistungen führen kann.

Die bisher beschriebenen Katalysatoren sind nicht voll befriedigend hinsichtlich der Standfestigkeit im technischen Betrieb, da sie bei Katalysatorbelastungen von 60 Nl Methacrolein je Stunde und Liter Katalysator und mehr Arbeitstemperaturen über 300°C benötigen und die Wirksamkeit schon bei Betriebszeiten von ca. 960 Stunden nachläßt. Sie sind auch technisch aus Gründen des Umweltschutzes in der Regel nicht problemlos herstellbar und/oder einsetzbar, da sie Arsen abgeben.

Im gegebenen Zusammenhang wurde gefunden, daß Arsen in zwei verschiedenen Bindungsarten im Katalysator vorliegen kann, wobei der eine Anteil bei den Herstellungstemperaturen über 250°C relativ flüchtig, der andere Anteil nicht bzw. nicht erkennbar flüchtig ist. Bei Arsenmengen über 0.5 Atomen je 12 Atomen Molybdän werden beträchtliche Mengen Arsenoxid während der thermischen Behandlung über die Gasphase aus den Reaktionsgefäßen ausgetragen. Es wird angenommen, daß der flüchtige Anteil, der auch aus Gründen des Umweltschutzes nicht tolerierbar ist, die Katalysatorleistung eher herabsetzt, während der nicht flüchtige Anteil die Wirksamkeit verbessert.

Es wurde des weiteren gefunden, daß die optimale Konzentration des Arsens von der Konzentration der anderen Komponenten, insbesondere des Vanadins, abhängig ist: je höher die Vanadin-Konzentration ist, umso niedriger muß die Arsenkonzentration sein, um eine Leistungsminderung des Katalysators zu vermeiden.

Es wurde ferner gefunden, daß bestimmte Mindestkonzentrationen des Vanadins und Phosphors notwendig sind, um ausreichende Standzeiten zu erreichen, daß die Aktivität der Katalysatoren jedoch bei zu hohen V-und P-Konzentrationen vermindert wird.

Es zeigte sich auch überraschend, daß bei einer Abstimmung der Konzentrationen der Hauptkomponenten Mo, P, V, Cs, As, Cu innerhalb sehr enger Grenzen und bei Anwendung bestimmter Herstellungsmethoden Katalysatoren erhalten werden, die sowohl eine hohe Selektivität als auch Aktivität und Standzeit aufweisen und die auch im Rahmen verschärfter Umweltschutzbestimmungen technisch hergestellt werden können.

Die Schriften DE-A-24 60 541, DE-A-33 46 259, US-A-4 051 179, US-A-3 010 434 und EP-A-43 100 betreffen ebenfalls Verfahren zur Herstellung von Methacrylsäure durch katalytische Gasphasenoxidation an Mo, P, V, Cs, As sowie teilweise Cu enthaltenden Katalysatoren, doch vermögen die in diesen Schriften offenbarten, auch die den Katalysatoren der vorliegenden Anmeldung am nächsten kommenden Katalysatoren, insbesondere hinsichtlich der Selektivität der Methacrylsäurebildung, nicht voll zu befriedigen.

Das erfindungsgemäße Verfahren zur Herstellung von Methacrylsäure durch Oxidation von Methacrolein in Sauerstoff und Wasserdampf enthaltenden Gasmischungen an Mo, P, V, Cs sowie As enthaltenden Katalysatoren ist daher dadurch gekennzeichnet, daß Katalysatoren eingesetzt werden, deren chemische Zusammensetzung wiedergegeben wird durch die allgemeine Formel

Mo₁₂PₐV_{b}Cs_{c}As_{d}CuₑX_{f}Y_{g}Oₓ,

wobei bedeuten
X = Zn, K, Rb, Ce, Nb, Fe, und/oder Zr
Y = Rh, Ag, S, Si, Ga und/oder Re
a = > 1.2 - 2.0
b = 0.4 - 1.2, vorzugsweise 0.5 - 1.1
c = 0.8 - 2.5, vorzugsweise > 1.25 - 2.0
d = > 0.18 - < 0.5
e = > 0.4 - 1.0
f = 0 - 0.8 für X = Zn, Ge, Nb, Fe, Zr oder
f = < 0,5 für X = K, Rb
g = 0 - 0.2
b + d = 0.75 - 1.4, vorzugsweise 0.9 - 1.4
x = die für die stöchiometrische Absättigung der Valenzen der übrigen Komponenten erforderliche Atomzahl.

Wie sich gezeigt hat, sind herausragend gut geeignete Katalysatoren solche, bei deren Herstellung Molybdän als Ammoniumsalz der Molybdän-, Phosphomolybdän- oder Phosphovanadatomolybdänsäure eingesetzt wird.

Wie sich weiter gezeigt hat, ist es von zusätzlichem Vorteil, insbesondere hinsichtlich der Aktivität, wenn bei der Herstellung der Katalysatoren das Cäsium als Acetat eingesetzt wird.

Darüber hinaus ist es vorteilhaft, Katalysatoren einzusetzen, bei deren Herstellung neben Cs auch Cu und ggf. kationische Elemente, soweit ihre Konzentration formelmäßig 0.4 Atome übersteigt, überwiegend als Acetate eingesetzt werden.

Das erfindungsgemäße Verfahren ist durch niedrige Arbeitstemperaturen bis unter 280°C über lange Betriebszeiten ausgezeichnet. Im Gegensatz zu bekannten Katalysatoren steigt die Aktivität im Laufe des Betriebes innerhalb der ersten 2 Monate ständig an. Außerdem werden nur geringe Mengen an organischen Nebenprodukten, insb. Essigsäure, gebildet, so daß die Reinisolierung der erzeugten Methacrylsäure wirtschaftlicher ist als bei den bekannten Verfahren.

Das erfindungsgemäße Verfahren arbeitet vorzugsweise bei Temperaturen, Raumbelastungen und Drücken, die einem Methacroleinumsatz im einfachen Durchgang von mehr als 50 mol-% und weniger als 70 mol-% entsprechen, im allgemeinen bei Temperaturen zwischen 250 und 350°C, Raumbelastungen zwischen 800 und 1800 Nl/l/h und Drücken von 1 bis 3 bar. Als Oxidationsmittel wird im allgemeinen Luft eingesetzt, der Sauerstoff kann jedoch auch in anderer Form, z.B. als Rein-Sauerstoff, zugeführt werden. Das Synthesegas enthält vorteilhaft Inertgase, wie N₂, CO und insbesondere Wasserdampf. In der bevorzugten Ausführungsform wird dem Reaktor ein Gemisch aus Methacrolein, Luft oder Sauerstoff und rückgeführtem Reaktorabgas, das nach Kondensation und/oder Auswaschen der höhersiedenden Wert- und Nebenprodukte hauptsächlich aus Kohlenoxiden, Stickstoff, nicht umgesetztem Sauerstoff und Methacrolein besteht und entsprechend den Trennbedingungen mit Wasserdampf gesättigt ist, eingespeist. Das molare Verhältnis von Methacrolein : O₂ : H₂O : Inertgasen beträgt im allgemeinen 1 : 1 - 3 : 2 - 20 : 3 - 30, vorzugsweise 1 : 1 - 3 : 3 - 10 : 7 - 18.

Das eingesetzte Methacrolein kann nach verschiedenen Verfahren hergestellt sein, z.B. durch Gasphasenoxidation von Isobutylen oder tert.-Butylalkohol. Bevorzugt wird Methacrolein verwendet, das durch Kondensation von Propanal mit Formaldehyd in Gegenwart von sek. Aminen und Säuren in flüssiger Phase nach den üblichen bekannten Verfahren hergestellt ist.

Die Umsetzung des Methacroleins wird im allgemeinen in Rohrbündelreaktoren durchgeführt, in denen der geformte Katalysator fest angeordnet ist. Auch die Oxidation in Wirbelschichtreaktoren ist möglich.

Das Reaktorabgas wird im allgemeinen nach indirekter und/oder direkter Kühlung bei Temperaturen zwischen meist 40 und 80°C mit Wasser gewaschen, wobei wäßrige Lösungen der Methacrylsäure erhalten werden, die geringe Mengen an Essig-, Malein-, Fumar-, Citracon-, Acryl- und Ameisensäure sowie Aldehyde, Ketone und nicht umgesetztes Methacrolein enthalten können. Die Methacrylsäure wird im allgemeinen aus der wäßrigen Lösung mit organischen Lösungsmitteln auf übliche Weise extrahiert und vom Lösungsmittel und den Nebenprodukten destillativ abgetrennt. Das nicht umgesetzte Methacrolein wird zum Teil aus dem wäßrigen Kondensat abdestilliert oder durch Strippen mit Luft bzw Inertgasen abgetrennt, zum Teil aus dem Abgas der Waschkolonne mit Wasser bei niedrigen Temperaturen ausgewaschen und wieder in den Reaktor zurückgeführt, vorzugsweise durch Verdampfen in den im Kreis geführten Teil des Abgases der Waschkolonne.

Die Katalysatoren können nach an sich bekannten Verfahrensweisen hergestellt werden, z.B. indem die Salze der Komponenten in Wasser gelöst und gemischt, getrocknet, geformt und schließlich einer Behandlung bei höheren Temperaturen unterworfen werden. Die Komponenten werden vorteilhaft in einer wasserlöslichen Form eingesetzt, Mo als Ammoniumsalz der Molybdän- oder Phosphomolybdänsäure, V als Ammoniummetavanadat oder Vanadyloxalat, P als o-Phosphorsäure oder Di-ammonium-Phosphat. Cäsium wird vorzugsweise als Acetat eingesetzt. Auch die übrigen kationischen Komponenten wie Alkalimetalle, Cu, Fe werden vorteilhaft als Acetate eingesetzt, soweit die Konzentration formelmäßig über 0.4 Atome je 12 Atome Mo beträgt. Der Einsatz von CsOH und höhere Nitratkonzentrationen sollten vermieden werden. Die wäßrige Lösung bzw. Suspension der Salze kann ggf. mit Essigsäure angesäuert werden, um einen pH-Wert unter 6.5 einzuhalten. Die Trocknung geschieht bei Temperaturen zwischen ca. 70 bis 180°C.

Die getrocknete Masse wird nach an sich ebenfalls bekannten Verfahrensweisen wie Tablettierung, Extrusion oder Strangpressen verformt, wobei Gleitmittel wie Graphit, Stearinsäure oder Stearate, Formhilfsmittel und Verstärkungsmittel wie Microfasern aus Glas, Asbest, SiC, Kaliumtitanat zugesetzt werden können. Die bevorzugte Teilchenform sind Hohlzylinder mit einem Außendurchmesser und einer Länge von 3 - 10 mm und einer Wandstärke von 1 - 3 mm, obwohl auch andere bekannte Formen wie Halbschalen, Sterne, Flachzylinder mit konvexen Stirnflächen verwendet werden können. Die Katalysatormasse kann auch als Pulver in Gegenwart eines Bindemittels oder als wäßrige Suspension auf vorgeformte Trägermaterialien zur Herstellung von sogenannten Schalenkatalysatoren nach bekannten Beschichtungsverfahren aufgetragen werden.

Die thermische Behandlung der Katalysatormasse erfolgt bei Temperaturen von 180°C bis maximal 480°C. Die Endtemperatur soll vorzugsweise 450°C nicht überschreiten und 350°C nicht unterschreiten. Die Calcinierung kann in Luft oder in inerter Atmosphäre z.B. Stickstoff, CO₂ durchgeführt werden. In einem bevorzugten Verfahren wird die thermische Behandlung in 2 oder mehr Temperaturstufen und mit ggf. wechselnder Gaszusammensetzung durchgeführt. Zunächst wird bei 200 bis 260°C in Luft in einer ersten Temperaturstufe, dann in N₂ mit einem O₂-Gehalt unter 5 Vol.-%, vorzugsweise von > 0 bis 3 Vol.-% bei 410 bis 460 °C in einer zweiten Temperaturstufe calciniert. Es kann vorteilhaft sein, den Katalysator anschließend bei Temperaturen von 350 bis 410 °C in Luft zu reoxidieren. Alternativ kann in der zweiten Temperaturstufe auch in Luft bei Temperaturen von 350 - 410°C calciniert werden.

Die Vorteile des erfindungsgemäßen Verfahrens gegenüber dem Stand der Technik gehen aus folgenden Beispielen hervor.

Bei den Mengenangaben bedeuten Teile Gewichtsteile, soweit nicht eine andere Definition angegeben ist.

### Beispiel 1

In 3 000 Teilen entionisiertes Wasser werden bei erhöhter Temperatur 530 Teile Ammoniumheptamolybdat-tetrahydrat, 35 Teile o-Phosphorsäure (85 %), 18 Teile einer 80 %igen o-Arsensäurelösung und 20 Teile Ammoniummetavanadat gelöst. Die Lösung wird mit einer Lösung von 51,2 Teilen Cäsiumacetat und 2 Teilen Eisessig in 45 Teilen Wasser versetzt. Schließlich werden 25 Teile Kupfer(II)acetat-monohydrat, gelöst in 750 Teilen Wasser, zugegeben. Der pH-Wert beträgt 6.0. Die erhaltene Suspension wird auf dem Wasserbad eingeengt und dann 16 Stunden bei 85°C getrocknet. Die erhaltene Trockenmasse wird gemahlen und nach Zumischen von 2 Gew.-% Graphitpulver zu Hohlzylindern mit einem Außendurchmesser von 7 mm, einer Länge von 6 mm und einem Lochdurchmesser von 3 mm verformt. Die Formlinge werden anschließend je 2 Stunden bei 210 und 230°C, 10 Stunden bei 250°C und schließlich 4 Stunden bei 400°C in einer Luftströmung von 80 Nl je Stunde und kg Katalysatormasse calciniert und anschließend auf eine Körnung von 3 mm zerkleinert. Der erhaltene Katalysator hat die formelmäßige Zusammensetzung

Mo₁₂V_{0.68}P_{1.21}Cs_{1.06}Cu_{0.5}As_{0.40}

75 Vol.-Teile des Katalysators werden in ein Salzbad-beheiztes Reaktionsrohr eingefüllt und dieses mit 95 000 Volumteilen einer Gasmischung aus 4.6 Vol.-% Methacrolein, 9 Vol.-% Sauerstoff, 25.5 Vol.-% Wasserdampf und 60.9 Vol.-% Stickstoff und Inertgasen beschickt. Im Reaktoraustrag wurden die Verbrennungsprodukte CO und CO₂ sowie die organischen Produkte und das nicht umgesetzte Methacrolein gaschromatographisch bzw. polarographisch bestimmt. Außerdem wurde die Gesamtmenge der gebildeten Säuren gemessen und nach Abzug der Säureäquivalenz der gebildeten Ameisen-, Essig-, Acryl-, Propion-, Malein-, Fumar- sowie Citraconsäure die gebildete Methacrylsäure. Die Ergebnisse sind in der Tabelle angegeben.

### Vergleichsbeispiel 1

Nach der Vorschrift des Beispiels 1 wird ein Katalysator der gleichen Zusammensetzung mit der Abweichung hergestellt, daß Cäsium als Cäsiumhydroxid CsOH und Kupfer als Kupfer(II)-Nitrat-Hydrat Cu(NO₃)₂·3 H₂O eingesetzt werden. Die Testergebnisse sind ebenfalls in der Tabelle angeführt.

### Beispiel 2

Nach der Vorschrift des Beispiels 1 wird ein weiterer Katalysator der gleichen Zusammensetzung mit der Abweichung hergestellt, daß Cäsium als Cäsiumnitrat CsNO₃ und Kupfer als Kupfer(II)-Nitrat-Hydrat Cu(NO₃)₂·H₂O eingesetzt werden. Die Testergebnisse sind in der Tabelle angegeben.

### Beispiel 3

In Analogie zur Vorschrift des Beispiels 2 wird ein Katalysator der Zusammensetzung

Mo₁₂P_{1.94}V_{1.0}Cs_{1.73}As_{0.2}Cu_{0.5}Rh_{0.008}

hergestellt und unter den Bedingungen des Beispiels 1 getestet. Die Ergebnisse sind wiederum in der Tabelle angegeben.

### Beispiel 4

Beispiel 3 wurde wiederholt mit der Änderung, daß die zweite Temperaturbehandlung bei 450°C in N₂-Atmosphäre mit 2 Vol.-% O₂ über eine Zeit von vier Stunden erfolgte. Anschließend wurde der Katalysator 2 Stunden in Luft bei 400°C reoxidiert. An der calcinierten Probe konnten keine NH₄-Gruppen nachgewiesen werden. Die Ergebnisse des katalytischen Tests sind in der Tabelle angegeben, sie unterscheiden sich nur unwesentlich von denen des Beispiels 3.

### Vergleichsbeispiele 2 und 3

Nach der Vorschrift des Beispiels 3 wurden 2 Katalysatoren mit der Änderung hergestellt, daß die Arsenmenge auf formelmäßig 0.53 (Vergleichsbeispiel 2) und 0.12 Atome (Vergleichsbeispiel 3) geändert wurde. Die Ergebnisse sind in der Tabelle angegeben. Beide Katalysatoren benötigen im Vergleich zum erfindungsgemäßen Katalysator des Beispiels 3 höhere Reaktionstemperaturen für einen vergleichbaren Umsatz und erzeugen mehr Nebenprodukte.

### Beispiele 5 bis 10

Nach der Vorschrift des Beispiels 2 werden weitere Katalysatoren hergestellt mit der Abweichung, daß die Mengenverhältnisse der Komponenten geändert und als zusätzliche Komponenten Rhodium als Rh(III)chloridhydrat, Nb, Zr, Ce, Re, als Oxide, K als Molybdat, die restlichen als Nitrate eingesetzt werden.
Beispiel 5: Mo₁₂P_{1.21}V_{0.68}Cs_{1.07}As_{0.21}Cu_{0.5}K_{0.1}Rh_{0.007}
Beispiel 6: Mo₁₂P_{1.94}V_{1.02}Cs_{1.73}As_{0.19}Cu_{0.5}Si_{0.02}Fe_{0.05}
Beispiel 7: Mo₁₂P_{1.59}V_{1.02}Cs_{1.43}As_{0.20}Cu_{0.5}Rb_{0.1}Rh_{0.009}
Beispiel 8: Mo₁₂P_{1.5}V_{0.6}Cs_{1.06}As_{0.19}Cu_{0.5}Zn_{0.3}Ga_{0.04}Re_{0.04}
Beispiel 9: Mo₁₂P_{1.21}V_{0.68}Cs_{1.06}As_{0.41}Cu_{0.5}Ce_{0.01}S_{0.03}Re_{0.1}
Beispiel 10: Mo₁₂P_{1.31}V_{0.7}Cs_{1.8}As_{0.41}Cu_{0.1}Ag_{0.01}Nb_{0.5}Zr_{0.1}

**Tabelle**

| Beispiel | Betriebsstunden | Badtemp. ^{°}C | Methacroleinumsatz, mol-% | Selektivität, mol-% | | |
|---|---|---|---|---|---|---|
| | | | | MAS | ES | CO/CO₂ |
| Bsp. 1 | 370 | 278 | 61.1 | 88.2 | 2.5 | 6.9 |
| | 1400 | 275 | 60.1 | 89.2 | 2.4 | 6.0 |
| Vgl.Bsp. 1 | 370 | 316 | 60.0 | 80.8 | 5.2 | 10.2 |
| Bsp. 2 | 370 | 293 | 62.4 | 88.7 | 2.9 | 6.2 |
| | 1400 | 286 | 60.5 | 89.0 | 2.5 | 5.7 |
| Bsp. 3 | 370 | 300 | 65.1 | 87.0 | 3.7 | 6.8 |
| Bsp. 4 | 370 | 300 | 61.3 | 86.4 | 3.8 | 7.0 |
| Vgl.Bsp. 2 | 370 | 319 | 56.0 | 82.3 | 4.3 | 9.8 |
| Vgl.Bsp. 3 | 370 | 317 | 57.4 | 84.4 | 4.1 | 8.7 |
| Bsp. 5 | 370 | 298 | 62.3 | 88.6 | 2.9 | 6.4 |
| Bsp. 6 | 370 | 305 | 60.2 | 87.1 | 3.7 | 6.5 |
| Bsp. 7 | 370 | 313 | 63.3 | 87.2 | 3.6 | 6.7 |
| Bsp. 8 | 370 | 303 | 65.1 | 86.9 | 3.2 | 6.3 |
| Bsp. 9 | 370 | 295 | 60.2 | 88.5 | 3.1 | 6.1 |
| Bsp. 10 | Es wurden in etwa die Ergebnisse des Bsp. 6 erzielt | | | | | |

### Vergleichsbeispiel 4

Zum Vergleich mit dem Stand der Technik wurde ein Katalysator nach der Vorschrift des Beispiels 88 der US-PS 4 596 784 mit der Zusammensetzung Mo₁₂P_{1.15}V_{0.35}Cs_{1.25}As_{1.3}Cu_{0.1} hergestellt, mit der Änderung, daß der Katalysator wie im Beispiel 1 zur vorliegenden Erfindung verformt wurde. Unter den Testbedingungen des Beispiels 1 wurde nach 370 Stunden erst bei Temperaturen von 340°C ein Umsatz von 58.2 mol-% erreicht. Die Selektivität der Bildung der Methacrylsäure, Essigsäure und der Kohlenoxide betrug 82.6, 4.4 und 11.2 mol-%.

## Patentansprüche

1. Verfahren zur Herstellung von Methacrylsäure durch Oxidation von Methacrolein in Sauerstoff und Wasserdampf enthaltenden Gasmischungen an Mo, P, V, Cs sowie As enthaltenden Katalysatoren, dadurch gekennzeichnet, daß Katalysatoren eingesetzt werden, deren chemische Zusammensetzung wiedergegeben wird durch die allgemeine Formel
Mo₁₂PₐV_{b}Cs_{c}As_{d}CuₑX_{f}Y_{g}Oₓ
wobei bedeuten
X = Zn, K, Rb, Ce, Nb, Fe und/oder Zr
Y = Rh, Ag, S, Si, Ga und/oder Re
a = > 1.2 - 2.0
b = 0.4 - 1.2
c = 0.8 - 2.5
d = > 0.18 - < 0.5
e = > 0.4 - 1.0
f = 0 - 0.8 für X = Zn, Ce, Nb, Fe, Zr oder
f = < 0.5 für X = K, Rb
g = 0 - 0.2
b + d = 0.75 - 1.4
x = die für die stöchiometrische Absättigung der Valenzen der übrigen Komponenten erforderliche Atomzahl.

2. Verfahren nach Anspruch 1, dadurch gekennzeichnet, daß Katalysatoren eingesetzt werden, bei deren Herstellung Molybdän als Ammoniumsalz der Molybdän-, Phosphomolybdän- oder Phosphovanadatomolybdänsäure eingesetzt wird.

3. Verfahren nach den Ansprüchen 1 bis 2, dadurch gekennzeichnet, daß Katalysatoren eingesetzt werden, bei deren Herstellung Cäsium als Acetat eingesetzt wird.

## Claims

1. A process for preparing methacrylic acid by oxidizing methacrolein in an oxygen- and steam-containing gas mixture over a catalyst which contains Mo, P, V, Cs and As, which comprises using a catalyst whose chemical composition is represented by the general formula
Mo₁₂PₐV_{b}Cs_{c}As_{d}CuₑX_{f}Y_{g}Oₓ,
where
X = Zn, K, Rb, Ce, Nb, Fe or Zr,
Y = Rh, Ag, S, Si, Ga or Re,
a = > 1.2-2.0,
b = 0.4-1.2,
c = 0.8-2.5,
d = > 0.18 - < 0.5,
e = > 0.4-1.0,
f = 0-0.8 for X = Zn, Ce, Nb, Fe, or Zr, or
f = < 0.5 for X = K or Rb,
g = 0-0.2,
b + d = 0.75-1.4, and
x = the number of atoms required for the stoichiometric saturation of the valences of the other components.

2. A process as claimed in claim 1, wherein the catalyst used is prepared using molybdenum in the form of the ammonium salt of molybdenic, phosphomolybdenic or phosphovanadatomolybdenic acid.

3. A process as claimed in claim 1 or 2, wherein the catalyst used is prepared using cesium in the form of the acetate.

## Revendications

1. Procédé de préparation de l'acide méthacrylique par l'oxydation de la méthacroléine dans des mélanges de gaz contenant de l'oxygène et de la vapeur d'eau, sur des catalyseurs contenant Mo, P, V, Cs comme aussi As, caractérisé en ce que l'on met en oeuvre des catalyseurs dont la composition chimique est rendue par la formule générale suivante
Mo₁₂PₐV_{b}Cs_{c}As_{d}CuₑX_{f}Y_{g}Oₓ
où
X = Zn, K, Rb, Ce, Nb, Fe et/ou Zr
Y = Rh, Ag, S, Si, Ga et/ou Re
a = > 1,2 - 2,0
b = 0,4 - 1,2
c = 0,8 - 2,5
d = > 0,18 - < 0,5
e = > 0,4 - 1,0
f = 0 - 0,8 pour X = Zn, Ce, Nb, Fe, Zr ou
f = > 0,5 pour X = K, Rb
g = 0 - 0,2
b + d = 0,75 - 1,4
x = le nombre atomique nécessaire pour la saturation stoechiométrique des valences des composants résiduels.

2. Procédé suivant la revendication 1, caractérisé en ce que l'on met en oeuvre des catalyseurs pour la préparation desquels on utilise le molybdène sous forme de sel d'ammonium de l'acide molybdique, de l'acide phosphomolybdique, ou de l'acide phosphovanadomolybdique.

3. Procédé suivant les revendications 1 et 2, caractérisé en ce que l'on met en oeuvre des catalyseurs pour la préparation desquels on utilise le césium sous forme d'acétate.
